# EUROPEAN PATENT SPECIFICATION

(11) **EP 3 438 260 B1**
(45) Date of publication and mention of the grant of the patent: **07.06.2023**
(21) Application number: 17775108.8
(22) Date of filing: 28.03.2017
(51) Int. Cl.: C12Q 1/6883

(54) **EYE DISEASE BIOMARKER**
BIOMARKER FÜR AUGENKRANKHEITEN
BIOMARQUEUR DE MALADIE OCULAIRE

(30) Priority: 28.03.2016 JP 2016064685
(43) Date of publication of application: 06.02.2019
(73) Proprietor: Osaka University, Suita-shi, Osaka 565-0871 (JP)
(72) Inventor: NISHIDA, Kohji, Suita-shi Osaka 565-0871 (JP); KUMANOGOH, Atsushi, Suita-shi Osaka 565-0871 (JP); HASHIDA, Noriyasu, Suita-shi Osaka 565-0871 (JP)
(74) Representative: Hoffmann Eitle
(86) International application number: PCT/JP2017/012689
(87) International publication number: WO 2017/170571

(56) References cited:
- EP-A1- 2 924 124
- WO-A1-2008/157747
- WO-A1-2014/038622
- WO-A1-2014/038622
- WO-A1-2014/080054
- WO-A1-2017/018382
- WO-A2-2013/119193
- WANG A.L. ET AL.: "Autophagy and exosomes in the aged retinal pigment epithelium: Possible relevance to drusen formation and age-related macular degeneration", PLOS ONE, vol. 4, no. 1, E4160, 8 January 2009 (2009-01-08), pages 1-13, XP055224997,
- GENDRON, SEBASTIEN P. et al.: "Mitochondrial DNA common deletion in the human eye: A relation with corneal aging", Mechanisms of Ageing and Development, vol. 133, 2012, pages 68-74, XP028403262,
- HAO, XIAO-DAN et al.: "Decreased Integrity, Content, and Increased Transcript Level of Mitochondrial DNA Are Associated with Keratoconus", PLOS One, vol. 11, no. 10, 26 October 2016 (2016-10-26), page e0165580, XP055429087,

## Description

### TECHNICAL FIELD

The present invention relates to a biomarker for eye disease that makes it possible to accurately evaluate corneal disease, conjunctival disease or central serous chorioretinopathy.

### BACKGROUND ART

As a method for evaluating susceptibility to a disease, presence or absence of the disease, the degree of progression of the disease or the like, a method using a biomarker is known. In a biomarker, a substance to be measured, such as protein, is present as a label in a living body fluid such as blood or urine, and the presence or degree of progression of a specific disease can be reflected in the concentration of the biomarker.

Various methods for evaluating a specific disease using a biomarker have been heretofore explored.

For example, Cited Document 1 discloses a biomarker for autoimmune disease which is composed of mitochondrial DNA contained in a living body fluid such as serum or urine. The biomarker for autoimmune disease has been completed by the present inventors with the finding that living body fluid of an autoimmune disease patient contains mitochondrial DNA at a high concentration.

Human lacrimal fluid includes moisture constituting 98% of the lacrimal fluid, and proteins such as albumin and globulin, and plays an important role in retaining moisture on the ocular surface and maintaining homeostasis. In various ocular diseases, a changes in osmotic pressure, and/or changes its composition such as protein concentration are observed in lacrimal fluid. Thus, lacrimal fluid is considered to acutely reflect states states of diseases not only in the eye but also in the whole body. It has been shown in previous studies that cytokine and chemokine proteins, etc. are involved in inflammation of the ocular surface, and in fact, detection of the inflammation with lacrimal fluid has been reported.

However, substances related to inflammation or any disease in ocular tissues, other than the above-mentioned substances detected as results of inflammation of the ocular surface, have not been identified. In addition, heretofore there has not been a substance identified as a biomarker for eye disease. On the ocular surface, corneal epithelial cells, conjunctival epithelial cells and the like are constantly repeat cell division and detachment, and these cell-derived components on the ocular surface are involved to stimulate a natural immunosensor, leading to occurrence of various inflammatory reactions. Thus, it is difficult to identify a substance that causes inflammation.

In addition, lacrimal fluid may be suitable as a test sample because of low invasiveness in sample collection, but when a substance is detected from lacrimal fluid, the sample amount is very small, so that it is difficult to identify and measure the substance with a conventional system. As described above, studies on a biomarker for eye disease have not sufficiently progressed.

### PRIOR ART DOCUMENT

### PATENT DOCUMENT

Patent Document 1: International Publication No. WO 2014/038622

### SUMMARY OF THE INVENTION

### PROBLEMS TO BE SOLVED BY THE INVENTION

A main object of the present invention is to provide a biomarker that makes it possible to apply lacrimal fluid as a sample and which makes it possible to conveniently and accurately evaluate corneal disease or conjunctival disease. In addition, a main object of the present invention is to provide a biomarker that makes it possible to conveniently and accurately evaluate central serous chorioretinopathy. In addition, the present invention provides examination methods using the biomarker.

### MEANS FOR SOLVING THE PROBLEMS

The present inventors have extensively conducted studies for achieving the above-mentioned object. As a result, the present inventors have found that a patient with corneal or conjunctival disease has a significantly larger amount of mitochondrial DNA (mtDNA) in lacrimal fluid as compared to a healthy control. In addition, the present inventors have found that since mtDNA is used, lacrimal fluid which is difficult to apply in a very small amount in a conventional technique can be used as a sample. Further, the present inventors have found that a patient with central serous chorioretinopathy has a significantly larger amount of mitochondrial DNA (mtDNA) in serum as compared to a healthy control. The present invention has been completed as a result of further conducting studies based on the above-described findings.

That is, the present invention provides the aspects described below. The invention is defined by the appended claims. In a first aspect, the present invention relates to a method for examining presence or absence of corneal or conjunctival diseases, the method including the steps of:
(i) measuring mitochondrial DNA in lacrimal fluid obtained from a test animal; and
(ii) detecting presence or absence of corneal or conjunctival diseases based on a result of the step (i), wherein the step (ii) is carried out based on whether a result of the step (i) which is obtained for the test animal shows a larger amount of mitochondrial DNA as compared to a result of the step (i) which is obtained for a normal control.

In a second aspect, the present invention relates to a method for examining presence or absence of central serous chorioretinopathy, the method including the steps of:
(i) measuring mitochondrial DNA in serum obtained from a test animal; and
(ii) detecting presence or absence of central serous chorioretinopathy based on a result of the step (i), wherein the step (ii) is carried out based on whether a result of the step (i) which is obtained for the test animal shows a larger amount of mitochondrial DNA as compared to a result of the step (i) which is obtained for a normal control.

### ADVANTAGES OF THE INVENTION

According to a biomarker of the present invention, corneal or conjunctival disease can be accurately diagnosed. In addition, in the present invention, there can be provided a system which can apply lacrimal fluid as a sample, and quantitatively evaluate corneal or conjunctival disease more conveniently. A result obtained based on the biomarker of the present invention contributes to optimization of a treatment method associated with the lesion of each patient. In addition, the present disclosure describes a diagnostic kit for corneal or conjunctival disease which includes the biomarker.

In addition, according to the biomarker of the present invention, central serous chorioretinopathy can be accurately evaluated. Further, the present disclosure describes a diagnostic kit for central serous chorioretinopathy which includes the biomarker.

### BRIEF DESCRIPTION OF THE DRAWINGS

Fig. 1 is a schematic diagram showing the secretion and metabolic pathway of exosomes.
Fig. 2 is a graph showing the results of Experimental Example 1, i.e. mtDNA levels in lacrimal fluid of a group of patients with ocular inflammation, corneal disease or retinal disease and a group of normal controls.
Fig. 3 is a graph showing the results of Experimental Example 2, i.e. mtDNA levels in lacrimal fluid of a group of patients with dry eye and a group of normal controls.
Fig. 4 is a graph showing the results of Experimental Example 3, i.e. the amounts of mtDNA in lacrimal fluid of a group of patients with keratoconus, limbal corneal stem cell deficiency or dry eye and a group of normal controls.
Fig. 5 is a graph showing the results of Experimental Example 4, i.e. mtDNA levels in serum of a group of patients with central serous chorioretinopathy and a group of normal controls.
Fig. 6 is a graph showing the results of Experimental Example 5, i.e. a relationship between a clinical finding (retinal OCT (optical coherence tomography) image) and a serum mitochondrial DNA concentration (left), and mtDNA levels in serum of a group of patients with central serous chorioretinopathy and a group of normal controls (right).
Fig. 7 is a diagram showing the results of Experimental Example 6, i.e. results of multi-variable analysis of clinical data (visual acuity, intraocular pressure, age, follow-up period, foveal choroid thickness, serous detachment and so on) and the concentration of mitochondrial DNA in serum for a patient with central serous chorioretinopathy.
Fig. 8 is a graph showing the results of Experimental Example 7, i.e. mtDNA levels in serum of a group of patients with keratoconus, a group of patients with dry eye, a group of patients with limbal corneal stem cell deficiency and a group of normal controls.
Fig. 9 is a graph showing the results of Experimental Example 8, i.e. results of measuring the concentration of IL-8 in the supernatant of a culture obtained by adding lacrimal fluid of a patient with keratoconus, a patient with limbal corneal stem cell deficiency, a patient with dry eye and a healthy control to CD14 positive monocytes derived from a healthy control, and culturing the resulting mixture.
Fig. 10 is a graph showing the results of Experimental Example 9, i.e. results of measuring the concentrations of IL-6, IL-8, MCP-1, VEGF and PDGF-AA in the supernatant of a culture obtained by adding lacrimal fluid of a patient with central serous chorioretinopathy and a healthy control to primary human retinal pigment epithelial cells, and culturing the resulting mixture.

### EMBODIMENTS OF THE INVENTION

### <1. Biomarker for Corneal or Conjunctival Disease>

### Biomarker

A biomarker for corneal or conjunctival disease according to a first embodiment of the present invention includes mitochondrial DNA (mtDNA) contained in lacrimal fluid. The lacrimal fluid of a patient with corneal or conjunctival disease contains mtDNA at a high concentration, and in the present invention, corneal or conjunctival disease is evaluated based on the concentration of mtDNA in lacrimal fluid.

The mtDNA is a circular double-stranded DNA present in the matrix of mitochondria which is a cell organelle. On mtDNA, cytochrome b and cytochrome c oxidase (COX) subunits I, II, III and II; subunits 1, 2, 3, 4, 4L, 5 and 6 of NADH dehydrogenase (NADH); and ATP synthetic enzyme (ATPase) sixth subunit and eighth subunit are encoded. Detection of mtDNA as a biomarker of the present invention can be performed by detecting DNAs of these genes encoded on mtDNA. One of the specific genes may be detected as a biomarker for corneal or conjunctival disease, or two or more thereof may be detected in combination, and used for evaluation of corneal or conjunctival disease. For example, COXIII, NADH dehydrogenase, cytochrome b and the like can be suitably used for detection of mtDNA which is a biomarker for corneal or conjunctival disease according to the present invention.

In addition, since the above-mentioned series of proteins are encoded on circular mtDNA, the behavior of mtDNA can be accurately detected by using as an indicator a DNA which encodes any of the proteins.

The source of lacrimal fluid is not particularly limited, and any animal can be used. More specific examples of the animal include humans, and mammals other than humans. Examples of the mammal other than humans include rodents such as mice, rats, hamsters and guinea pigs, and laboratory animals such as rabbits; domestic animals such as pigs, cows, goats, horses and sheep; pets such as dogs and cats; primates such as humans, monkeys, orangutans and chimpanzees. In the present invention, the source of lacrimal fluid is preferably a primate, more preferably a human.

The living body fluid to be used in the biomarker of corneal or conjunctival disease according to the present invention is lacrimal fluid. The lacrimal fluid contains mtDNA. Since the biomarker for corneal or conjunctival disease according to the present invention has mtDNA as a label, mtDNA can be quantified even in a drop sample such as lacrimal fluid. In addition, lacrimal fluid is collected with a low level of invasiveness. Thus, in the biomarker of the present invention, lacrimal fluid is used, and therefore evaluation can be performed more conveniently and accurately as compared to conventional techniques.

In the present invention, lacrimal fluid is obtained by collecting the lacrimal fluid directly from the eye with a dropper, a glass capillary or the like. In addition, the lacrimal fluid in the present invention may be one obtained by washing the eye with a solution, and recovering the solution after washing. As the solution after washing the eye, mention is made of, for example, a PBS (phosphate buffer solution) used as a wiping liquid for the lower palpebral conjunctiva, and recovered. Specifically, 30 µL of PBS is taken with a micropipette, and a subject is made to turn up. While an observation is made with a slit-lamp microscope, the PBS is dropped onto the lower palpebral conjunctiva of the subject, and mtDNA on the conjunctiva is immediately recovered as a conjunctiva wiping liquid.

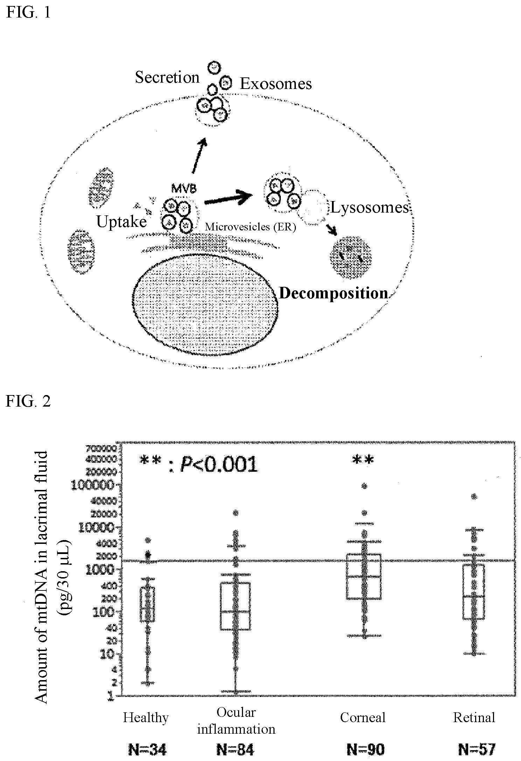

More preferably, mtDNA localized in an extracellular membrane vesicle fraction in lacrimal fluid is used as the biomarker of the present invention. The extracellular membrane vesicle contains exosomes and microvesicles called ectosomes, and it is more preferable that mtDNA localized in an exosome fraction is used as the biomarker of the present invention. The exosome is a vesicle composed of a lipid bilayer membrane with a diameter of 50 to 200 nm, and is known to include encapsulating mRNA, microRNA, and various proteins present in the cytoplasm, and have various biological activities depending on donor cells. The exosome buds toward a secretory vesicle arising from a late endosome, forms a multivesicular body (MVB), and is transported to the cell surface. MVB is fused with a cell membrane, so that the exosome which is a content of the MVB is released outside the cell. Normally, MVB is transported not only to the cell surface, but also to a lysosome, and fused with the lysosome, so that the content thereof is decomposed (see Fig. 1 for the above).

It is considered that since mtDNA is present in such an extracellular membrane vesicle having a lipid bilayer membrane, particularly in the exosome, mtDNA is protected from being decomposed by a DNA degrading enzyme (DNase) present outside the cell, and exists stably. Thus, by using mtDNA present in the exosome as a biomarker, corneal or conjunctival disease can be further accurately evaluated. In addition, it is known that exosomes are also contained in lacrimal fluid, and it may be possible to establish a low-invasive or noninvasive evaluation system by isolating exosomes from lacrimal fluid, and measuring mtDNA contained in the lacrimal fluid.

The method for separating exosomes is not particularly limited as long as a sample usable for detection of mtDNA is obtained, and one of previously known methods can be appropriately selected. Exosomes can be separated by ultracentrifugation, but more conveniently, exosomes can be separated using a commercially available kit, and specific examples of the kit include ExoQuick^{™} Exosome precipitation solution and Exoquick-TC (Both manufactured by System Biosciences Company).

In addition, mtDNA in lacrimal fluid is also contained in fractions other than exosomes, and may be present in a free state as exposed nucleic acids, or associated with proteins.

Detection of mtDNA is performed by preparing a polynucleotide from the lacrimal fluid or exosome fraction, and carrying out an amplification reaction with a primer using the polynucleotide as a template, or subjecting the polynucleotide to a hybridization reaction using a probe. From the viewpoint of stability and accuracy of evaluation, the polynucleotide to be prepared is preferably DNA. Preparation of the polynucleotide from lacrimal fluid or exosome fractions can be performed by appropriately using a known method, and examples of the method include methods of phenol extraction and ethanol precipitation, and methods using glass beads. More conveniently, the polynucleotide can be prepared using a commercially available DNA extraction reagent or a DNA extraction kit. Specific examples of the DNA extraction kit include DNA Extractor SP Kit (manufactured by Wako Pure Chemical Industries, Ltd.).

Detection of DNA corresponding to the aforementioned protein encoded on mtDNA can be performed by a method appropriately selected from previously known methods. For detection, a polynucleotide serving as a probe or an oligonucleotide serving as a primer is normally used for measurement of the expression level or amplification of the protein. As the primer, mention is made of an oligonucleotide having a unique sequence capable of being specifically hybridized to at least a part of a target gene to amplify the gene. In addition, as the probe, mention is made of a polynucleotide having a unique sequence that is specifically hybridized to at least a part of a target gene.

These primers or probes can be designed and synthesized in accordance with a previously known method based on sequence information of a target gene, which is obtained using a program and database such as BLAST or FASTA. The length of the base sequence of the primer is normally 16 to 32 bp, preferably 18 to 30 bp, more preferably 20 to 24 bp. Specific examples of the primer set for specifically amplifying the cytochrome b gene include primer sets which are used in examples as described later, and are expressed by SEQ ID NOS: 1 and 2. In addition, specific examples of the primer set for specifically amplifying the COXIII gene include 5'-ATGACCCACCAATCACATGC-3' (forward primer: SEQ ID NO: 3); and 5'-ATCACATGGCTAGGCCGGAG-3' (reverse primer: SEQ ID NO: 4). In addition, specific examples of the primer set for specifically amplifying the NADH gene include 5'-ATACCCATGGCCAACCTCCT-3' (forward primer: SEQ ID NO: 5); and 5'-GGGCCTTTGCGTAGTTGTAT-3' (reverse primer: SEQ ID NO: 6).

Detection of mtDNA can be performed based on an amplification product obtained by a gene amplification reaction using the primer as described above, or a hybrid product obtained by a hybridization reaction using a probe.

The method for amplification of a target gene is not particularly limited, and a previously known method can be employed. Examples thereof include amplification of DNA/RNA by a polymerase chain reaction (PCR), more specifically RT-PCR, Nested PCR, real-time PCR, competitive PCR, TaqMan PCR and Direct PCR. In addition, a modified PCR method such as a LAMP (Loop-mediated isothermal Amplification) method, an ICAN (Isothermal and Chimeric primer-initiated Amplification of Nucleic Acids) method, or a RCA (Rolling Circle Amplification) method may be used.

The method for detection of an amplification product is not particularly limited as long as it is possible to determine whether the product is a desired polynucleotide or not. For example, whether or not a polynucleotide with a predetermined size is amplified can be checked by agarose gel electrophoresis. In addition, the amplification product can be detected by labeling deoxynucleotide triphosphate (dNTP) taken in the amplification product in the process of the amplification reaction, and measuring a label substance of dNTP taken in the amplification product. Examples of the label substance include fluorescent substances such as fluorescein (FITC), sulforhodamine (SR) and tetramethylrhodamine (TRITC); luminescent substances such as luciferin; and radioactive isotopes such as 32P, 35S and 121I. Alternatively, the amplification product may be quantitatively detected by an intercalator method using SYBR Green or the like.

In addition, the length of the base sequence of the probe used for detection of mtDNA is normally 20 to 250 bp, preferably 20 to 100 bp, more preferably 20 to 50 bp. In addition, the probe that is hybridized to the polynucleotide is not required to have a perfect complementary sequence as long as it can be hybridized to at least a part of the polynucleotide, and detected.

For the hybridization reaction using the probe, conditions under which the probe is specifically hybridized to a target polynucleotide (stringent conditions), for example conditions under which DNAs having homology of 90% or more are hybridized to each other, and DNAs having lower homology are not hybridized to each other, can be appropriately set based on previously known conditions. As stringent conditions, for example, washing is performed at a temperature of 40°C to 70°C, a sodium concentration of 150 to 900 mM and a pH of 6 to 8, more specifically washing is performed at 50°C with 2 × SSC (300 mM NaCl and 30 mM citric acid) and 0.1 vol% SDS.

In addition, if necessary, the probe may be labeled using a fluorescent substance such as fluorescein (FITC) or sulforhodamine (SR), tetramethylrhodamine (TRITC); a luminescent substance such as luciferin; a radioactive isotope such as 32P, 35S or 121I; an enzyme such as alkali phosphatase or horseradish peroxidase; a label substance such as biotin. By detecting the target substance in a previously known method based on the type of each label substance after the hybridization reaction, a hybridization product can be detected.

An animal (preferably a human) with corneal or conjunctival disease has a significantly larger amount of mtDNA contained in lacrimal fluid (or in exosomes), i.e. a biomarker of the present invention, as compared to a group of normal controls. Here, the group of normal controls refers to healthy test animals (preferably humans) which do not have corneal or conjunctival disease and have no inflammatory symptoms.

In the present invention, corneal disease is a disease in which the cornea is damaged or deformed, or bacteria or virus infects the cornea to cause inflammation, leading to development of the disease. Examples of the corneal disease include keratoconus, limbal corneal stem cell deficiency, dry eye, diffuse superficial keratitis, corneal epithelial erosion, bacterial corneal infection, corneal ulcer, interstitial keratitis, keratomalacia, corneal opacity and corneal degeneration.

The conjunctival disease is a disease in which conjunctival inflammation occurs, leading to development of the disease, and examples of the conjunctival disease include conjunctivitis, allergic conjunctivitis, epidemic keratoconjunctivitis, pharyngoconjunctival fever, subconjunctival hemorrhage, lithiasis conjunctivae, Stevens-Johnson's syndrome and ocular pemphigoid. In particular, the biomarker of the present invention is suitably used as a biomarker for keratoconus, dry eye, or limbal corneal stem cell deficiency.

As described above, the biomarker of the present invention makes it possible to conveniently and accurately evaluate corneal or conjunctival disease.

### Diagnostic Kit (not forming part of the invention)

A diagnostic kit for corneal or conjunctival disease includes a reagent capable of detecting the mtDNA. When detection of mtDNA is performed based on a gene or gene fragment encoded on mtDNA, a primer that specifically amplifies the gene or gene fragment, or a probe which is specifically hybridized to the gene or gene fragment is included as a reagent capable of detecting mtDNA. Specific examples of the reagent included in the kit include primers shown in SEQ ID NOS: 1 to 6. These primers and probes are as described in the section of "Biomarker" for corneal or conjunctival disease.

Further, the reagent capable of detecting mtDNA may contain a buffer solution, a salt, a stabilizer, a preservative and the like, and may be formulated in accordance with a previously known method. Further, in addition to the reagent, the diagnostic kit may contain a label substance, a label substance detecting agent, a reaction diluting solution, a standard antibody, a buffer solution, a solubilizing agent, a cleaning agent, a reaction stopping solution, a control sample and the like which may be required to perform the detection of mtDNA.

In the kit, for example, a probe for detecting mtDNA can be used with the probe immobilized on an insolubilized carrier. Therefore, the diagnostic kit may also include an insolubilized carrier. The material of the insolubilized carrier is not particularly limited as long as it does not hinder detection of mtDNA, and examples thereof include polystyrene, polyethylene, polypropylene, polyester, polyacrylonitrile, polyvinyl chloride, fluororesin, crosslinked dextran, polysaccharide, paper, silicon, glass, metal and agarose. Two or more of these materials may be used in combination. The shape of the insolubilized carrier may be any of, for example, a microplate shape, a tray shape, a spherical shape, a fiber shape, a rod shape, a disk shape, a container shape, a cell shape, a test tube shape and the like.

For example, a probe that can be specifically hybridized to a gene encoded on mtDNA can be immobilized on the insolubilized carrier to obtain a DNA chip to be used for detection of corneal or conjunctival disease. Immobilization of the probe on the insolubilized carrier can be performed in accordance with a previously known method. In addition, when probes for mtDNA are immobilized on a carrier at different concentrations and at equal intervals, and hybridized to mtDNA, mtDNA can be semi-quantitatively detected.

### Evaluation method based on the amount of mtDNA in lacrimal fluid

The present invention provides a method for examining presence or absence of corneal or conjunctival disease using mtDNA in lacrimal fluid, which is the biomarker, the method including the steps of:
(i) measuring mitochondrial DNA in lacrimal fluid obtained from a test animal; and
(ii) detecting presence or absence of corneal or conjunctival disease based on a result of the step (i).

Measurement of mtDNA in lacrimal fluid as described in the step (i) can be performed in accordance with the procedure described in the section of "Biomarker". In addition, the detection step described in the step (ii) is carried out based on whether a result of the step (i) which is obtained for the test animal shows a larger amount of mitochondrial DNA as compared to a result of the step (i) which is obtained for a group of normal controls. Here, the group of normal controls is as described in the section of "Biomarker".

In the detection step described in the step (ii), the possibility that the test animal has corneal or conjunctival disease is determined to be high when the amount of mitochondrial DNA in lacrimal fluid obtained from the test animal is larger than that in the group of normal controls. Further, the possibility that the test animal has corneal or conjunctival disease is determined to be very high when the amount of mitochondrial DNA in lacrimal fluid obtained from the test animal is markedly larger than that in the group of normal controls. In the present invention, the phrase "the amount of mitochondrial DNA is larger than that in the group of normal controls" means that it is equal to or greater than the average value of the amount of mitochondrial DNA in lacrimal fluid of the group of normal controls + 2 × SD (standard deviation), and the phrase "the amount of mitochondrial DNA is markedly larger than that in the group of normal controls" means that it is equal to or greater than the average value of the amount of mitochondrial DNA in lacrimal fluid of the group of normal controls + 4 × SD. The average value and SD of the amount of mitochondrial DNA in lacrimal fluid of the group of normal controls can be determined by measuring the amount of mitochondrial DNA in lacrimal fluid of, for example, a group of 24 or more normal controls. In addition, for the amount of mitochondrial DNA in lacrimal fluid of the group of normal controls, the present inventors have found that the average value + 2 × SD is 648.1 pg (picogram)/30 µL, and the average value + 4 × SD is 854.5 pg /30 µL. Therefore, it can be determined that "the amount of mitochondrial DNA is larger than that in normal controls" when the amount of mitochondria in lacrimal fluid is 648.1 pg/30 µL or more, and "the amount of mitochondrial DNA is markedly larger than that in normal controls" when the amount of mitochondria in lacrimal fluid is 854.5 pg/30 µL or more.

In addition, examples of the test animal include humans, and mammals other than humans. Examples of the mammal other than humans include rodents such as mice, rats, hamsters and guinea pigs, and laboratory animals such as rabbits; domestic animals such as pigs, cows, goats, horses and sheep; pets such as dogs and cats; primates such as humans, monkeys, orangutans and chimpanzees. In the present invention, the test animal is preferably a primate, more preferably a human.

Further, evaluation based on the biomarker of the present invention can be utilized not only for examination of presence or absence of corneal or conjunctival disease, but also for a method for prediction of susceptibility to corneal or conjunctival disease, a method for detection of severity, a method for prediction of prognosis, a method for screening of a therapeutic agent, and a method for differential diagnosis of corneal or conjunctival disease.

When susceptibility to corneal or conjunctival disease is evaluated based on the biomarker of the present invention (method for prediction of susceptibility to corneal or conjunctival disease), the susceptibility is predicted based on the result of measuring mitochondrial DNA in lacrimal fluid obtained from a test animal as in the case of the method for examining presence or absence of corneal or conjunctival disease. The susceptibility is predicted in accordance with the following criterion: the test animal is susceptible to corneal or conjunctival disease when the amount of mitochondrial DNA is larger than that in a group of normal controls, and the test animal is particularly susceptible to corneal or conjunctival disease when the amount of mitochondrial DNA is markedly larger than that in a group of normal controls.

When the severity of corneal or conjunctival disease is evaluated based on the biomarker of the present invention (method for detection of severity of cornea or conjunctival disease), the severity is detected based on the result of measuring mitochondrial DNA in lacrimal fluid obtained from a test animal as in the case of the method for examining presence or absence of corneal or conjunctival disease. The severity is detected in accordance with the following criterion: there is the possibility that the test animal has severe corneal or conjunctival disease when the amount of mitochondrial DNA is larger than that in a group of normal controls, and the possibility is particularly high that the test animal has particularly severe corneal or conjunctival disease when the amount of mitochondrial DNA is markedly larger than that in a group of normal controls. Alternatively, the severity is detected based on the following criteria: the test animal has severe corneal or conjunctival disease when the biomarker value detected from the test animal is higher than the standard value of the biomarker in the corneal or conjunctival disease.

When the prognosis of corneal or conjunctival disease is evaluated based on the biomarker of the present invention (method for prediction of prognosis of corneal or conjunctival disease), the prognosis is predicted based on the result of measuring mitochondrial DNA in lacrimal fluid obtained from a test animal as in the case of the method for examining presence or absence of corneal or conjunctival disease. The prognosis is predicted in accordance with the following criterion: there is the possibility that the test animal has poor prognosis of corneal or conjunctival disease when the amount of mitochondrial DNA is larger than that in a group of normal controls, and the possibility is high that the test animal has poor prognosis of corneal or conjunctival disease when the amount of mitochondrial DNA is markedly larger than that in a group of normal controls. Alternatively, the prognosis is evaluated based on the following criterion: the test animal has poor prognosis of the disease when the biomarker value detected from the test animal is higher than the standard value of the biomarker in the corneal or conjunctival disease.

When screening of a therapeutic agent effective for corneal or conjunctival disease is performed based on the biomarker of the present invention, the screening is performed based on the result of measuring mitochondrial DNA in lacrimal fluid obtained from a test animal with corneal or conjunctival disease, which has been given the therapeutic agent, as in the case of the method for examining presence or absence of corneal or conjunctival disease. The screening is performed based on the following criterion: the therapeutic agent is effective when the amount of mitochondrial DNA is smaller than that in a test animal with corneal or conjunctival disease, which has not been given the therapeutic agent.

When differential diagnosis in corneal or conjunctival disease is performed, i.e. differentiation from similar disease is performed, based on the biomarker of the present invention, the differentiation from similar disease is performed based on the result of measuring mitochondrial DNA in lacrimal fluid obtained from a test animal as in the case of the method for examining presence or absence of corneal or conjunctival disease. The differentiation from similar disease is performed based on the following criterion: there is the possibility that the test animal has corneal or conjunctival disease when the amount of mitochondrial DNA is larger than the standard value of the biomarker in the disease to be compared.

In any of these methods, a correlation diagram between the amount of mtDNA in lacrimal fluid and presence or absence of corneal or conjunctival disease, severity of corneal or conjunctival disease, susceptibility to corneal or conjunctival disease, prognosis of corneal or conjunctival disease, or the like may be prepared beforehand, followed by applying the amount of mtDNA in lacrimal fluid of a test animal to the correlation diagram to perform evaluation.

Based on these evaluation methods, disease can be evaluated at an individual level, so that the treatment method can be optimized according to an individual disease condition.

### <2. Biomarker of Central Serous Chorioretinopathy>

### Biomarker

The second invention is a biomarker for central serous chorioretinopathy which includes mitochondrial DNA contained in serum.

The biomarker for central serous chorioretinopathy according to the present invention includes mitochondrial DNA (mtDNA) in serum. The serum of a patient with central serous chorioretinopathy contains mitochondrial DNA (mtDNA) at a high concentration, and in the biomarker of the present invention, central serous chorioretinopathy is evaluated based on the concentration of mtDNA in serum.

The mtDNA is as described in the section of "Biomarker for Corneal or Conjunctival Disease".

The source of serum is not particularly limited, and any animal can be used. More specific examples of the animal include humans, and mammals other than humans. Examples of the mammal other than humans include rodents such as mice, rats, hamsters and guinea pigs, and laboratory animals such as rabbits; domestic animals such as pigs, cows, goats, horses and sheep; pets such as dogs and cats; primates such as humans, monkeys, orangutans and chimpanzees. In the present invention, the source of serum is preferably a primate, more preferably a human.

In the present invention, serum is obtained by removing blood cells, and blood coagulation factors such as fibrinogen (factor I), prothrombin (factor II), factor V and factor VIII from blood (whole blood). The method for obtaining serum is not particularly limited, and serum can be obtained according to a method employed in clinical examinations and the like. For example, serum can be obtained as a supernatant obtained after blood is left standing, or a supernatant obtained by subjecting blood to centrifugal separation. In addition, prior to the detection of the biomarker for central serous chorioretinopathy according to the present invention, a pretreatment utilizing filtration with a filter or a column may be performed for removing contaminants such as serum proteins as necessary. Examples of the serum protein include albumin, transferrin, haptoglobin, transthyretin, α1 antitrypsin, α2 macroglobulin, α1-acid glycoprotein, immunoglobulin G (IgG), immunoglobulin A (IgA), immunoglobulin M (IgM), complement C3, apolipoprotein AI and apolipoprotein AII.

More preferably, mtDNA localized in an extracellular membrane vesicle fraction in serum is used as the biomarker of the present invention. The extracellular membrane vesicle contains exosomes and microvesicles called ectosomes, and it is more preferable that mtDNA localized in an exosome fraction is used as the biomarker of the present invention. The mtDNA and exosomes localized in an extracellular membrane vesicle fraction in the present invention are as described in the section of "Biomarker for Corneal or Conjunctival Disease".

It is considered that since mtDNA is present in such an extracellular membrane vesicle having a lipid bilayer membrane, particularly in the exosome, mtDNA is protected from being decomposed by a DNA degrading enzyme (DNase) present outside the cell, and exists stably. Thus, by using mtDNA present in the exosome as a biomarker, central serous chorioretinopathy can be further accurately evaluated. In addition, it is known that exosomes are also contained in a large amount in serum, and it may be possible to establish a low-invasive or noninvasive evaluation system by isolating exosomes from serum, and measuring mtDNA contained in the serum.

The method for separation of exosomes and the method for detection of mtDNA in the present invention are as described in the section of "Biomarker for Corneal or Conjunctival Disease" except that as living body fluid serum is used in place of lacrimal fluid.

An animal (preferably a human) with central serous chorioretinopathy has a significantly larger amount of mtDNA contained in serum (or in exosomes), i.e. a biomarker of the present invention, as compared to a group of normal controls. Here, the group of normal controls refers to healthy test animals (preferably humans) which do not have central serous chorioretinopathy and have no inflammatory symptoms.

As described above, the biomarker of the present invention makes it possible to conveniently and accurately evaluate central serous chorioretinopathy.

### Diagnostic Kit

The use of a diagnostic kit for central serous chorioretinopathy according to the present invention includes a reagent capable of detecting the mtDNA. When detection of mtDNA is performed based on a gene or gene fragment encoded on mtDNA, a primer that specifically amplifies the gene or gene fragment, or a probe which is specifically hybridized to the gene or gene fragment is included as a reagent capable of detecting mtDNA. Specific examples of the reagent included in the kit include primers shown in SEQ ID NOS: 1 to 6. These primers and probes are as described in the section of "Biomarker" for corneal or conjunctival disease and central serous chorioretinopathy.

Other reagents that may be contained as reagents capable of detecting mtDNA are as described in the section of "Diagnostic Kit" for corneal or conjunctival disease. Further, in the kit, for example, a probe for detecting mtDNA may be used with the probe immobilized on an insolubilized carrier. The immobilization of the probe on the insolubilized carrier is as described in the section of "Diagnostic Kit" for corneal or conjunctival disease.

### Evaluation method based on the amount of mtDNA in serum

The present invention provides a method for examining presence or absence of central serous chorioretinopathy using mitochondrial DNA in serum, which is the biomarker, the method including the steps of:
(i) measuring mitochondrial DNA in serum obtained from a test animal; and
(ii) detecting presence or absence of central serous chorioretinopathy based on a result of the step (i).

Measurement of mitochondrial DNA in serum obtained from a test animal as described in the step (i) can be performed in accordance with the procedure described in the section of "Biomarker". In addition, the detection step described in the step (ii) is carried out based on whether a result of the step (i) which is obtained for the test animal shows a larger amount of mitochondrial DNA as compared to a result of the step (i) which is obtained for normal controls. Here, the group of normal controls is as described in the section of "Biomarker".

In the detection step described in the step (ii), the possibility that the test animal has central serous chorioretinopathy is determined to be high when the amount of mitochondrial DNA in serum obtained from the test animal is larger than that in the group of normal controls. Further, the possibility that the test animal has central serous chorioretinopathy is determined to be very high when the amount of mitochondrial DNA in serum obtained from the test animal is markedly larger than that in the group of normal controls. In the present invention, the phrase "the amount of mitochondrial DNA is larger than that in the group of normal controls" means that it is equal to or greater than the average value of the amount of mitochondrial DNA in serum of the group of normal controls + 2 × SD (standard deviation), and the phrase "the amount of mitochondrial DNA is markedly larger than that in the group of normal controls" means that it is equal to or greater than the average value of the amount of mitochondrial DNA in serum of the group of normal controls + 4 × SD. The average value and SD of the amount of mitochondrial DNA in serum of the group of normal controls can be determined by measuring the amount of mitochondrial DNA in serum of, for example, a group of 24 or more normal controls. In addition, for the amount of mitochondrial DNA in serum of the group of normal controls (humans), the present inventors have found that the average value + 2 × SD is 13.1 ng/mL, and the average value + 4 × SD is 16.9 ng/mL. Therefore, it can be determined that "the amount of mitochondrial DNA is larger than that in normal controls" when the amount of mitochondria in serum is 13.1 ng/mL or more, and "the amount of mitochondrial DNA is markedly larger than that in normal controls" when the amount of mitochondria in serum is 16.9 ng/mL or more.

In addition, examples of the test animal include humans, and mammals other than humans. Examples of the mammal other than humans include rodents such as mice, rats, hamsters and guinea pigs, and laboratory animals such as rabbits; domestic animals such as pigs, cows, goats, horses and sheep; pets such as dogs and cats; primates such as humans, monkeys, orangutans and chimpanzees. In the present invention, the test animal is preferably a primate, more preferably a human.

Further, evaluation based on the biomarker of the present invention can be utilized not only for examination of presence or absence of central serous chorioretinopathy, but also for a method for prediction of susceptibility to central serous chorioretinopathy, a method for detection of severity, a method for prediction of prognosis, a method for screening of a therapeutic agent, and a method for differential diagnosis of central serous chorioretinopathy.

When susceptibility to central serous chorioretinopathy is evaluated based on the biomarker of the present invention (method for prediction of susceptibility to central serous chorioretinopathy), the susceptibility is predicted based on the result of measuring mitochondrial DNA in serum obtained from a test animal as in the case of the method for examining presence or absence of central serous chorioretinopathy. The susceptibility is predicted in accordance with the following criterion: the test animal is susceptible to central serous chorioretinopathy when the amount of mitochondrial DNA is larger than that in a group of normal controls, and the test animal is particularly susceptible to central serous chorioretinopathy when the amount of mitochondrial DNA is markedly larger than that in a group of normal controls.

When the severity of central serous chorioretinopathy is evaluated based on the biomarker of the present invention (method for detection of severity of central serous chorioretinopathy), the severity is detected based on the result of measuring mitochondrial DNA in serum obtained from a test animal as in the case of the method for examining presence or absence of central serous chorioretinopathy. The severity is detected in accordance with the following criterion: there is the possibility that the test animal has severe central serous chorioretinopathy when the amount of mitochondrial DNA is larger than that in a group of normal controls, and the possibility is high that the test animal has severe central serous chorioretinopathy when the amount of mitochondrial DNA is markedly larger than that in a group of normal controls. Alternatively, the severity is detected based on the following criteria: the disease is severe when the biomarker value detected from the test animal is higher than the standard value of the biomarker in the disease.

When the prognosis of central serous chorioretinopathy is evaluated based on the biomarker of the present invention (method for prediction of prognosis of central serous chorioretinopathy), the prognosis is predicted based on the result of measuring mitochondrial DNA in serum obtained from a test animal as in the case of the method for examining presence or absence of central serous chorioretinopathy. The prognosis is predicted in accordance with the following criterion: there is the possibility that the test animal has poor prognosis of central serous chorioretinopathy when the amount of mitochondrial DNA is larger than that in a group of normal controls, and the possibility is high that the test animal has poor prognosis of central serous chorioretinopathy when the amount of mitochondrial DNA is markedly larger than that in a group of normal controls. Alternatively, the prognosis is evaluated based on the following criterion: the test animal has poor prognosis of the disease when the biomarker value detected from the test animal is higher than the standard value of the biomarker in the disease.

When screening of a therapeutic agent effective for central serous chorioretinopathy is performed based on the biomarker of the present invention, the screening is performed based on the result of measuring mitochondrial DNA in serum obtained from a test animal with central serous chorioretinopathy, which has been given the therapeutic agent, as in the case of the method for examining presence or absence of central serous chorioretinopathy. The screening is performed based on the following criterion: the therapeutic agent is effective when the amount of mitochondrial DNA is smaller than that in a test animal with central serous chorioretinopathy, which has not been given the therapeutic agent.

When differential diagnosis in central serous chorioretinopathy is performed, i.e. differentiation from similar disease is performed, based on the biomarker of the present invention, the differentiation from similar disease is performed based on the result of measuring mitochondrial DNA in serum obtained from a test animal as in the case of the method for examining presence or absence of central serous chorioretinopathy. The differentiation from similar disease is performed based on the following criterion: the test animal has central serous chorioretinopathy when the amount of mitochondrial DNA is larger than the standard value of the biomarker in the disease to be compared.

In any of these methods, a correlation diagram between the amount of mtDNA in serum and presence or absence of central serous chorioretinopathy, severity of central serous chorioretinopathy, susceptibility to central serous chorioretinopathy, prognosis of central serous chorioretinopathy, or the like may be prepared beforehand, followed by applying the amount of mtDNA in serum of a test animal to the correlation diagram to perform evaluation.

Based on these evaluation methods, disease can be evaluated at an individual level, so that the treatment method can be optimized according to an individual disease condition.

### EXAMPLES

Hereinafter, the present invention will be described by way of test examples, but the present invention is not limited to these test examples.

All the tests were conducted with the approval of the Ethics Committee of Osaka University Hospital.

### (Experimental Example 1)

A patients having ocular inflammation (uveitis), corneal disease or retinal disease (age-related macular degeneration) was used as a subject. The amount of mitochondrial DNA (mtDNA) in lacrimal fluid of the subject was measured and evaluated in accordance with the following method. The amount of mtDNA in lacrimal fluid of each of healthy controls as a group of normal controls was measured under the same conditions. The subjects included 84 ocular inflammation patients, 90 corneal disease patients, 57 retinal disease patients and 34 normal persons (healthy controls).

### Preparation of Lacrimal Fluid Sample

As a lacrimal fluid sample, 30 µL of PBS (phosphate buffer solution) used as a wiping liquid for the lower palpebral conjunctiva, and recovered was used. Specifically, 30 µL of PBS was taken with Pipetman, and a subject was made to turn up. While an observation was made with a slit-lamp microscope, the PBS was dropped onto the lower palpebral conjunctiva of the subject, and mtDNA on the conjunctiva was immediately recovered as a conjunctiva wiping liquid. The thus-obtained liquid was used as the lacrimal fluid sample.

### Separation of DNA in Lacrimal Fluid

Using QIAamp DNA mini Kit from Qiagen K.K., lacrimal fluid DNA was prepared from the obtained lacrimal fluid (5 µl) in accordance with the attached protocol. Specifically, 5 µl of the lacrimal fluid sample was digested with 415 µl of a proteolytic enzyme (56°C, 10 minutes), DNA was precipitated with 100 vol% ethanol, and the DNA was purified using a purification column attached to the kit. The obtained lacrimal fluid DNA was resuspended in DNase-free water (20 µl).

### Real Time PCR

Using SYBR Premix Ex Taq (Perfect Real Time) (manufactured by Takara Bio Inc.), real time PCR by an intercalator method using SYBR Green I was performed by ABI PRISM 7700 (Life Technologies Japan) in accordance with the attached protocol. The PCR conditions are as follows.
Stage 1 (1 cycle): 95°C for 30 seconds;
Stage 2 (40 cycles): 95°C for 5 seconds and 60°C for 30 seconds; and
Stage 3 (1 cycle): 95°C for 15 seconds, 60°C for 1 minute and 95°C for 15 seconds

Using purified DNA derived from lacrimal fluid of healthy controls, a real time standard curve was prepared for determining the mtDNA concentration. Further, a sample which did not produce a PCR product even after completion of 40 cycles of the PCR reaction (i.e. a sample in which emission of fluorescence by SYBR Green was not observed) was considered as being "undetectable".

Primers used for detection of mitochondrial DNA are shown below.

### (Cytochrome b)

Forward primer (SEQ ID NO: 1):
   5'-ATGACCCCAATACGCAAAAT-3'
Reverse primer (SEQ ID NO: 2):
   5'-CGAAGTTTCATCATGCGGAG-3'

The total amount of mtDNA in the lacrimal fluid was determined in the following manner: fluorescein Na was dropped at a known concentration to the ocular surface beforehand, the fluorescence amount of fluorescein Na in the recovered lacrimal sac washing solution was measured before and after the recovery to determine the dilution ratio, and the amount of lacrimal fluid on the whole ocular surface was estimated. The obtained mtDNA level was statistically examined by the Steel test method. The results are shown in the graph in Fig. 2.

Fig. 2 is a graph showing mtDNA levels in lacrimal fluid of a group of patients with various eye diseases, and a group of normal controls. The graph in Fig. 2 shows that the mtDNA level in lacrimal fluid in the ocular inflammation group and the retinal disease group is not significantly higher than that in the group of normal controls, but the mtDNA level in lacrimal fluid in the corneal disease group is significantly higher than that in the group of normal controls (P < 0.001). This indicates that corneal disease is associated with an increase in mtDNA level in lacrimal fluid, and thus mtDNA in lacrimal fluid can serve as a biomarker for corneal disease.

### (Experimental Example 2)

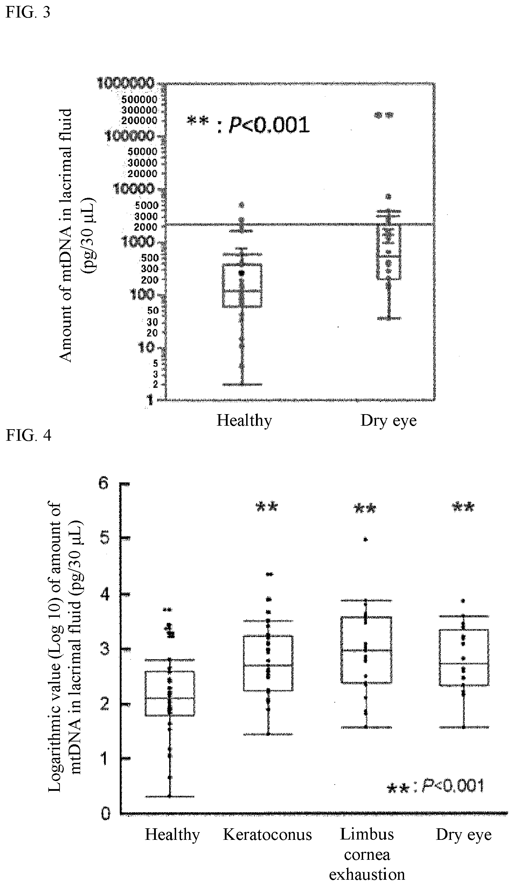

The amount of mtDNA in lacrimal fluid of each of dry eye patients (10 patients) was evaluated using the same method as in Example 1. The amount of mtDNA in lacrimal fluid of each of healthy controls (20 persons) as a group of normal controls was measured under the same conditions. The obtained mtDNA level was statistically analyzed by the Student's t test method. The results are shown in Fig. 3.

Fig. 3 is a graph showing mtDNA levels in lacrimal fluid of a group of dry eye patients and a group of normal controls. The graph in Fig. 3 shows that the mtDNA level in lacrimal fluid in the group of dry eye patients is significantly higher than that in the group of normal controls (P < 0.001). This indicates that a dry eye patient has an increased mtDNA level in lacrimal fluid as compared to a group of normal persons (healthy controls), and thus mtDNA in lacrimal fluid can serve as a biomarker for dry eye.

### (Experimental Example 3)

The expression level of mtDNA in lacrimal fluid of a subject with keratoconus, limbal corneal stem cell deficiency or dry eye was measured and evaluated by the same method as in Example 1. The expression level of mtDNA in lacrimal fluid of each of healthy controls as a group of normal controls was measured under the same conditions. The subjects included 39 keratoconus patients, 29 limbal corneal stem cell deficiency patients, 20 dry eye patients and 34 healthy controls. The expression level of mtDNA was statistically examined by the Steel test method after a relative value obtained based on a calibration curve was logarithmically processed. The results are shown in Fig. 4.

Fig. 4 is a graph showing expression levels of mtDNA in lacrimal fluid of a group of patients with keratoconus, limbal corneal stem cell deficiency or dry eyes and a group of normal controls. The graph in Fig. 4 shows that the expression level of mtDNA in lacrimal fluid in each of the groups of patients with keratoconus, limbal corneal stem cell deficiency and dry eye is significantly higher than that in the group of normal controls (P < 0.001). This indicates that keratoconus limbal corneal stem cell deficiency or dry eye is associated with an increase in mtDNA level in lacrimal fluid, and thus mtDNA in lacrimal fluid can serve as a biomarker for keratoconus, limbal corneal stem cell deficiency or dry eye.

### (Experimental Example 4)

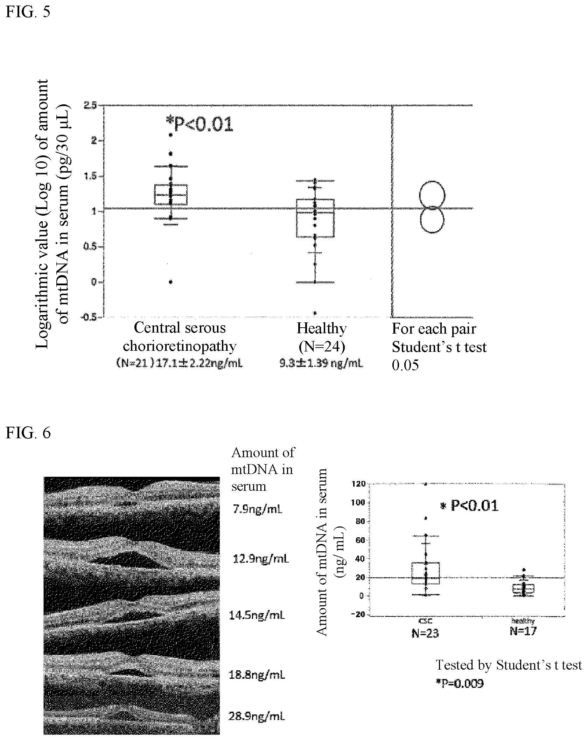

Serum was taken from each of patients (21 patients) with central serous chorioretinopathy, and the amount of mtDNA in the serum was measured and evaluated by the method shown below. Serum was taken from each of healthy controls (24 persons) as a group of normal controls, and the amount of mtDNA in the serum was measured under the same conditions. The results are shown in the graph in Fig. 5.

### Separation of Serum DNA

Using QIAamp DNA mini Kit from Qiagen Co. Ltd., serum DNA was prepared from the serum (100 µl) in accordance with the attached protocol. Specifically, 100 µl of the serum sample was digested with 320 µl of a proteolytic enzyme (56°C, 10 minutes), DNA was precipitated with 100 vol% ethanol, and the DNA was purified using a purification column attached to the kit. The obtained serum DNA was resuspended in DNase-free water (20 µl).

### Real Time PCR

Using SYBR Premix Ex Taq (Perfect Real Time) (manufactured by Takara Bio Inc.), real time PCR by an intercalator method using SYBR Green I was performed by ABI PRISM 7700 (Life Technologies Japan) in accordance with the attached protocol. The PCR conditions are as follows.
Stage 1 (1 cycle): 95°C for 30 seconds;
Stage 2 (40 cycles): 95°C for 5 seconds and 60°C for 30 seconds; and
Stage 3 (1 cycle): 95°C for 15 seconds, 60°C for 1 minute and 95°C for 15 seconds

Using purified DNA derived from a whole cell lysate of peripheral blood mononuclear cells (PBMC) of healthy controls, a real time standard curve was prepared for determining the mtDNA concentration. Further, a sample which did not produce a PCR product even after completion of 40 cycles of the PCR reaction (i.e. a sample in which emission of fluorescence by SYBR Green was not observed) was considered as being "undetectable".

Primers used for detection of mitochondrial DNA are shown below.

### (Cytochrome b)

Forward primer (SEQ ID NO: 1):
   5'-ATGACCCCAATACGCAAAAT-3'
Reverse primer (SEQ ID NO: 2):
   5'-CGAAGTTTCATCATGCGGAG-3'

The obtained mtDNA level was statistically examined by the Student's t test method. The results are shown in the graph in Fig. 5.

Fig. 5 is a graph showing mtDNA concentrations in serum of a group of patients with central serous chorioretinopathy and a group of normal controls. The graph in Fig. 5 shows that the mtDNA concentration in serum in the group of patients with central serous chorioretinopathy is significantly higher than that in the group of normal controls (P < 0.01). It is apparent that since the mtDNA concentration in serum in the group of patients with central serous chorioretinopathy is higher than that in the group of normal controls, mtDNA in serum can be used as a biomarker for central serous chorioretinopathy.

### (Experimental Example 5)

In this experimental example, the number n in the test in Experimental Example 4 was increased, and the same analysis was performed. Specifically, serum was taken from each of patients (23 patients) with central serous chorioretinopathy, and the amount of mtDNA in the serum was measured in the same manner as in Experimental Example 4. Serum was taken from each of healthy controls (24 persons) as a group of normal controls, and the amount of mtDNA in the serum was measured under the same conditions. The obtained value was statistically examined by the Student's t test method.

The results are shown in Fig. 6. The left diagram in Fig. 6 shows clinical findings (OCT (optical coherence tomography) images of the retina) and the results of the mitochondrial DNA concentration in serum for six patients with central serous chorioretinopathy. The right diagram in Fig. 6 shows the results of measuring the amount of mtDNA in serum in the patient group (CSC) and the group of normal controls (Healthy). The results show that the mtDNA concentration in serum in the group of patients with central serous chorioretinopathy was higher than that in the group of normal controls. As is evident from the left diagram in Fig. 6, the amount of mtDNA in serum tended to increase as serous retinal detachment progressed (the amount of subretinal fluid increased). These results show that mtDNA in serum can be used as a biomarker for central serous chorioretinopathy.

### (Experimental Example 6)

For examining a correlation between the mitochondrial DNA concentration in serum and clinical data of the visual acuity, eye pressure, age, follow-up period, foveal choroid thickness, serous detachment and the like for patients (23 patients) with central serous chorioretinopathy for which the mitochondrial DNA concentration was measured in Experimental Example 5, multi-variable analysis was performed using statistical analysis software JMP 13 (SAS Institute Inc.).

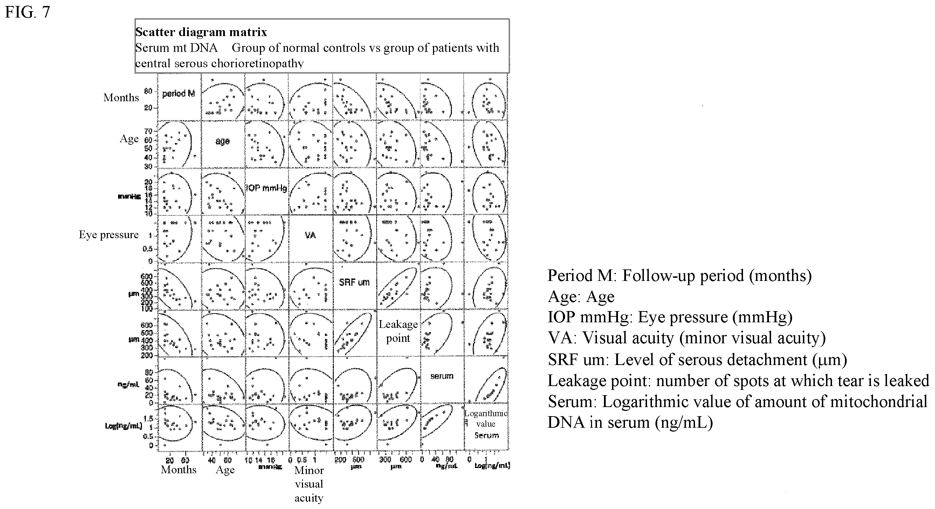

The results are shown in Fig. 7. The disease condition progresses as the level of serous detachment in Fig. 7 becomes higher. Since there was a positive correlation between the level of serous detachment and the mitochondrial DNA concentration in serum, it was revealed that the more severe the disease condition, the higher the mitochondrial DNA concentration in blood. In addition, as the follow-up period in Fig. 7 becomes longer, the disease condition becomes stable due to release from a stress state. Since there was a negative correlation between the follow-up period and the mitochondrial DNA concentration in serum, it was confirmed that the mitochondrial DNA concentration in blood decreased when the disease condition was stable. That is, these results revealed that not only there was a mere correlation between central serous chorioretinopathy and the mitochondrial DNA concentration in serum, but also the level of the mitochondrial DNA concentration was significantly correlated with the activity of the disease.

### (Experimental Example 7)

The expression level of mitochondrial DNA in lacrimal fluid of each of subjects with keratoconus (33 subjects, 42 eyes; KC), dry eye (10 subjects, 20 eyes; Dry eye) or limbal corneal stem cell deficiency (19 subjects, 39 eyes; LSCD) was measured and evaluated by the same method as in Example 1. In addition, serum was taken from the subject, and the amount of mitochondrial DNA in the serum was measured in the same manner as in Experimental Example 4. Lacrimal fluid and serum were taken from each of healthy controls (20 persons, 40 eyes) as a group of normal controls, and the amount of mitochondrial DNA in the serum was measured under the same conditions. The obtained value was statistically examined by the Student's t test method.

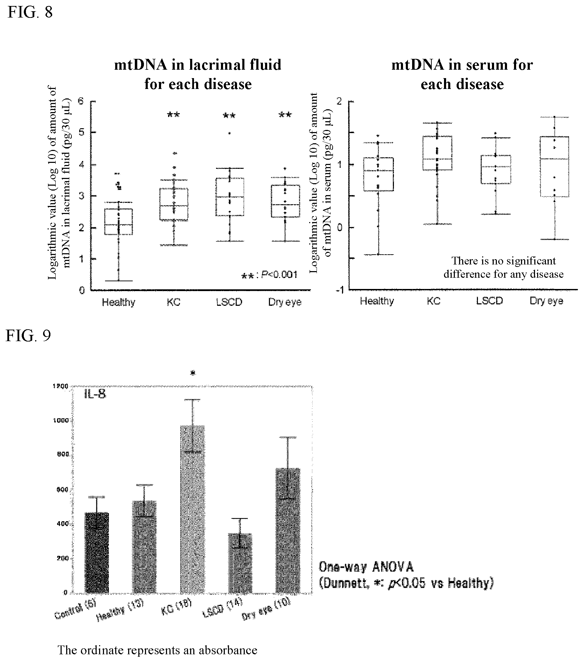

The results are shown in Fig. 8. There was no significant difference in the amount of mtDNA in serum between the patient with corneal disease and the healthy control, but the amount of mitochondrial DNA in lacrimal fluid in each of the patients with keratoconus limbal corneal stem cell deficiency and dry eye was significantly larger than that in the healthy control. Of course, these diseases were not developed with disorders of the whole body, and had lesions only in eyes, and it was revealed that local events in eyes increased the mitochondrial DNA concentration in lacrimal fluid.

### (Experimental Example 8)

60 µl of a cell suspension containing healthy control-derived CD14 positive monocytes at a concentration of 1 × 10⁶ cells/ml was put in a microplate, and cultured for 1.5 hours, 10 µl of lacrimal fluid taken from each of patients with keratoconus (18 patients; KC), limbal corneal stem cell deficiency (14 patients; LSCD) or dry eye (10 patients; Dry eye) was then added, and the resulting mixture was cultured for 4 hours. After the culture, the culture supernatant was recovered, and the concentration of IL-8 in the culture supernatant was measured by ELISA. As a control, a similar test was conducted without adding lacrimal fluid, and a similar test was conducted using lacrimal fluid taken from each of healthy controls (13 persons; Healthy) as a group of normal controls. The lacrimal fluid was taken by the method shown in Experimental Example 1.

In this test, cytokine is secreted in the culture supernatant to increase the concentration when lacrimal fluid contains a substance that stimulates inflammatory cells.

The results are shown in Fig. 9. The concentration of IL-8 that is involved in the real invasion of inflammatory cells was increased only with lacrimal fluid of patients with keratoconus. It was revealed that the amount of mitochondrial DNA in lacrimal fluid was increased in keratoconus which had not been previously considered to be associated with inflammation, and in keratoconus, inflammatory cells were attracted through IL-8 recruiting the inflammatory cells, leading to development of the disease condition.

### (Experimental Example 9)

60 µl of a cell suspension containing primary human retinal pigment epithelial cells (Lonza Japan Co., Ltd.) at a concentration of 1 × 10⁶ cells/ml was put in a microplate, and cultured for 1.5 hours, 10 µl of serum taken from each of patients with central serous chorioretinopathy (23 patients; CSC) was then added, and the resulting mixture was cultured for 4 hours. After the culture, the culture supernatant was recovered, and the concentration of each of IL-6, IL-8, MCP-1, VEGF and PDGF-AA in the culture supernatant was measured by ELISA. As a control, a similar test was conducted using PBS in place of lacrimal fluid, and a similar test was conducted using lacrimal fluid taken from each of healthy controls (24 persons; Healthy) as a group of normal controls.

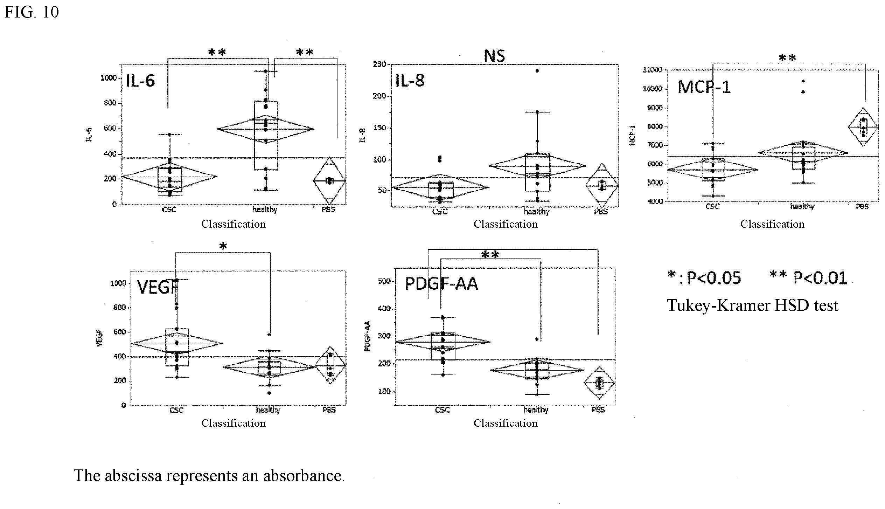

The results are shown in Fig. 10. When serum of the patient with central serous chorioretinopathy was added, the concentrations of IL-6, IL-8, and MCP-1 involved in inflammation were not increased, and therefore it was confirmed that central serous chorioretinopathy was not an inflammation. On the other hand, when serum of the patient with central serous chorioretinopathy was added, the concentrations of VEGF and PDGF-AA involved in enhancement of vascular permeability were increased. That is, a factor generated in central serous chorioretinopathy was found to be involved in pathogenesis with vascular permeability enhanced by increasing the concentrations of VEGF and PDGF-AA.
SEQ ID NO: 1 is a forward primer for cytochrome b.
SEQ ID NO: 2 is a reverse primer for cytochrome b.
SEQ ID NO: 3 is a forward primer for COXIII.
SEQ ID NO: 4 is a reverse primer for COXIII.
SEQ ID NO: 5 is a forward primer for NADH dehydrogenase.
SEQ ID NO: 6 is a reverse primer for NADH dehydrogenase.

## Claims

1. Use of a kit which includes a reagent capable of detecting mitochondrial DNA for detecting mitochondrial DNA in serum for diagnosing central serous chorioretinopathy.

2. A method for examining presence or absence of corneal or conjunctival diseases, the method comprising the steps of:
(i) measuring mitochondrial DNA in lacrimal fluid obtained from a test animal; and
(ii) detecting presence or absence of corneal or conjunctival diseases based on a result of the step (i), wherein the step (ii) is carried out based on whether a result of the step (i) which is obtained for the test animal shows a larger amount of mitochondrial DNA as compared to a result of the step (i) which is obtained for a normal control.

3. A method for examining presence or absence of central serous chorioretinopathy, the method comprising the steps of:
(i) measuring mitochondrial DNA in serum obtained from a test animal; and
(ii) detecting presence or absence of central serous chorioretinopathy based on a result of the step (i), wherein the step (ii) is carried out based on whether a result of the step (i) which is obtained for the test animal shows a larger amount of mitochondrial DNA as compared to a result of the step (i) which is obtained for a normal control.

4. Use of mitochondrial DNA contained in lacrimal fluid as a biomarker for diagnosing corneal or conjunctival disease.

5. The use according to claim 4, wherein the corneal or conjunctival disease is keratoconus, limbal corneal stem cell deficiency or dry eye.

6. Use of mitochondrial DNA contained in serum as a biomarker for diagnosing central serous chorioretinopathy.

## Patentansprüche

1. Verwendung eines Kits, das ein Reagenz mit der Fähigkeit zum Nachweis mitochondrialer DNA enthält, zum Nachweis mitochondrialer DNA in Serum zur Diagnose zentraler seröser Chorioretinopathie.

2. Verfahren zur Untersuchung des Vorhandenseins oder der Abwesenheit von Hornhaut- oder Bindehauterkrankungen, wobei das Verfahren die folgenden Schritte umfasst:
(i) Messen der mitochondrialen DNA in der von einem Testtier erhaltenen Tränenflüssigkeit; und
(ii) Detektieren des Vorhandenseins oder der Abwesenheit von Hornhaut- oder Bindehauterkrankungen basierend auf einem Ergebnis des Schritts (i),
wobei der Schritt (ii) basierend darauf durchgeführt wird, ob ein Ergebnis des Schritts (i), das für das Testtier erhalten wird, eine größere Menge an mitochondrialen DNA im Vergleich zu einem Ergebnis des Schritts (i), das für eine normalen Kontrolle erhalten wird zeigt.

3. Verfahren zur Untersuchung des Vorhandenseins oder der Abwesenheit einer zentralen serösen Chorioretinopathie, wobei das Verfahren die folgenden Schritte umfasst:
(i) Messen der mitochondrialen DNA in dem von einem Testtier erhaltenen Serum; und
(ii) Detektieren des Vorhandenseins oder der Abwesenheit von zentraler seröser Chorioretinopathie basierend auf einem Ergebnis des Schritts (i),
wobei der Schritt (ii) basierend darauf durchgeführt wird, ob ein Ergebnis des Schritts (i), das für das Testtier erhalten wird, eine größere Menge an mitochondrialen DNA im Vergleich zu einem Ergebnis des Schritts (i), das für eine normalen Kontrolle erhalten wird zeigt.

4. Verwendung der in der Tränenflüssigkeit enthaltenen mitochondrialen DNA als Biomarker für die Diagnose von Erkrankungen der Hornhaut oder der Bindehaut.

5. Verwendung gemäß Anspruch 4, wobei die Erkrankung der Hornhaut oder der Bindehaut Keratokonus, limbaler Hornhautstammzellmangel oder trockenes Auge ist.

6. Verwendung von mitochondrialer DNA im Serum als Biomarker für die Diagnose von zentraler seröser Chorioretinopathie.

## Revendications

1. Utilisation d'un kit qui inclut un réactif capable de détecter de l'ADN mitochondrial pour détecter de l'ADN mitochondrial dans un sérum pour diagnostiquer une choriorétinopathie séreuse centrale.

2. Procédé de vérification de la présence ou de l'absence de maladies cornéennes ou conjonctivales, le procédé comprenant les étapes consistant à :
(i) mesurer de l'ADN mitochondrial dans un fluide lacrymal obtenu à partir d'un animal de laboratoire ; et
(ii) détecter la présence ou l'absence de maladies cornéennes ou conjonctivales sur la base d'un résultat de l'étape (i), dans lequel l'étape (ii) est effectuée sur la base de si un résultat de l'étape (i) qui est obtenu pour l'animal de laboratoire montre une plus grande quantité d'ADN mitochondrial qu'un résultat de l'étape (i) qui est obtenu pour un contrôle normal.

3. Procédé de vérification de la présence ou de l'absence d'une choriorétinopathie séreuse centrale, le procédé comprenant les étapes consistant à :
(i) mesurer de l'ADN mitochondrial dans un sérum obtenu à partir d'un animal de laboratoire ; et
(ii) détecter la présence ou l'absence d'une choriorétinopathie séreuse centrale sur la base d'un résultat de l'étape (i), dans lequel l'étape (ii) est effectuée sur la base de si un résultat de l'étape (i) qui est obtenu pour l'animal de laboratoire montre une plus grande quantité d'ADN mitochondrial qu'un résultat de l'étape (i) qui est obtenu pour un contrôle normal.

4. Utilisation d'ADN mitochondrial contenu dans un fluide lacrymal comme biomarqueur pour diagnostiquer une maladie cornéenne ou conjonctivale.

5. Utilisation selon la revendication 4, dans laquelle la maladie cornéenne ou conjonctivale est un kératocône, un déficit en cellules souches cornéennes limbiques ou une sécheresse oculaire.

6. Utilisation d'ADN mitochondrial contenu dans un sérum comme biomarqueur pour diagnostiquer une choriorétinopathie séreuse centrale.
